# EUROPEAN PATENT APPLICATION

(11) **EP 4 767 981 A1**
(43) Date of publication of application: **01.07.2026**
(21) Application number: 25176342.1
(22) Date of filing: 14.05.2025
(51) Int. Cl.: A61B 34/20, A61B 34/00, A61B 34/30, A61B 90/00, A61B 34/10

(54) **SURGICAL SYSTEM AND DECOMPRESSION SYSTEM**

(30) Priority: 24.12.2024 TW 113150320
(71) Applicant: Point Robotics Medtech Inc., 30261 Zhubei City, Hsinchu Co (TW)
(72) Inventor: WANG, Ren-Jeng, 30261 Zhubei City, Hsinchu County (TW); CHAN, Hsin, 30261 Zhubei City, Hsinchu County (TW); SHIH, Yun-Shuo, 30261 Zhubei City, Hsinchu County (TW); TIAN, Yong-Hou, 30261 Zhubei City, Hsinchu County (TW); WENG, Jiun-Yu, 30261 Zhubei City, Hsinchu County (TW); CHEN, Shih-Yuan, 30261 Zhubei City, Hsinchu County (TW); CHEN, Yen-Jung, 30261 Zhubei City, Hsinchu County (TW); TSAI, Sheng-Chou, 30261 Zhubei City, Hsinchu County (TW)
(74) Representative: Viering, Jentschura & Partner mbB Patent- und Rechtsanwälte

(57) **Abstract**

A surgical system (SS) and a decompression system (1) are disclosed. The surgical system (SS) includes a surgical navigation module (10), a robotic arm (11), a surgical tool (12), a cannular scope (13), detection markers (14), a navigation image capture device (15), and a display device (DD). The surgical tool (12) includes a force sensor (120) configured to measure current force data. The display device (DD) allows a user to plan a predetermined surgical path (SP), and displays an anatomy model of a patient, the predetermined surgical path (SP), and an image of a surgical site captured by at least one camera (134). In a system operation program, the surgical navigation module (10) determines a drive mode of the robotic arm (11) based on a current force vector (v1), a cannula axis vector (u1), and the predetermined surgical path (SP). When the user operates the surgical tool (12), the surgical tool (12) is moved along the predetermined surgical path (SP).

## Description

### FIELD OF THE INVENTION

The present invention relates to a system, and more particularly to a surgical system and a decompression system.

### BACKGROUND OF THE INVENTION

Spinal decompression surgery (also called spinal decompression) is a procedure used to treat a herniated disc in the spine or other spinal problems. During the procedure, a surgeon makes a small incision on the back of the patient near the affected area and uses surgical instruments through the incision to repair or remove portions of the disc for relieving pressure on the nerves.

In order to improve the accuracy and safety of the surgery, a cannular scope is inserted into the body of the patient through the incision during the surgery, and images of internal organs are captured through the endoscopic lens to provide a clear field of view to surgeons for facilitating the surgery.

However, during the surgery, since spatial positions of the surgical instruments relative to the cannular scope cannot be accurately determined, the surgical instruments are prone to collide with the cannular scope, thus damaging the cannular scope.

### SUMMARY OF THE INVENTION

In response to the above-referenced technical inadequacies, the present invention provides a surgical system and a decompression system.

In order to solve the above-mentioned problems, one of the technical aspects adopted by the present invention is to provide a surgical system. The surgical system includes a surgical navigation module, a robotic arm, a surgical tool, a cannular scope, a plurality of detection markers, a navigation image capture device, and a display device. The robotic arm is electrically connected to the surgical navigation module. The surgical tool is disposed on the robotic arm. The surgical tool includes a force sensor, and the force sensor is configured to measure current force data on the surgical tool. The cannular scope is disposed on a movable support arm. The cannular scope has a cannula axis and at least one camera. The plurality of detection markers are respectively disposed on the surgical tool, the cannular scope, and a patient. The navigation image capture device is electrically connected to the surgical navigation module and configured to capture images of the plurality of detection markers. The display device is electrically connected to the surgical navigation module for allowing a user to plan a predetermined surgical path. The display device displays an anatomy model of the patient, the predetermined surgical path, and an image of a surgical site captured by the at least one camera. The surgical navigation module is configured to calculate and obtain a cannula axis vector of the cannular scope based on the plurality of detection markers, and calculate and obtain a current force vector of the surgical tool based on the current force data. In a system operation program, after the surgical tool is operated and located on the predetermined surgical path aligned with the cannula axis, the surgical navigation module is configured to determine a drive mode of the robotic arm based on the current force vector, the cannula axis vector, and the predetermined surgical path, so that when the user operates the surgical tool, the surgical tool is moved along the predetermined surgical path.

In order to solve the above-mentioned problems, the other of the technical aspects adopted by the present invention is to provide a decompression system. The decompression system includes a surgical navigation module, a robotic arm, a surgical tool, a cannular scope, a plurality of detection markers, and an image capture device. The robotic arm is electrically connected to the surgical navigation module. The surgical tool includes a force sensor. The force sensor is configured to measure current force data on the surgical tool. The cannular scope has a cannula axis. The plurality of detection markers are respectively disposed on the surgical tool, the cannular scope, and a patient. The image capture device is electrically connected to the surgical navigation module and configured to capture images of the plurality of detection markers. The surgical navigation module is configured to calculate and obtain a cannula axis vector of the cannular scope based on marker positions of the plurality of detection markers, and calculate and obtain a current force vector of the surgical tool based on the current force data. In a system operation program, after the surgical tool is operated and located on the predetermined surgical path aligned with the cannula axis, the surgical navigation module is configured to determine a drive mode of the robotic arm based on the current force vector, the cannula axis vector, and the predetermined surgical path, so that when the user operates the surgical tool, the surgical tool is moved along the predetermined surgical path.

These and other aspects of the present invention will become apparent from the following description of the embodiment taken in conjunction with the following drawings and their captions, although variations and modifications therein may be affected without departing from the spirit and scope of the novel concepts of the invention.

### BRIEF DESCRIPTION OF THE DRAWINGS

The described embodiments may be better understood by reference to the following description and the accompanying drawings, in which:
FIG. 1 is a schematic view of configurations of a surgical system and a decompression system according to one embodiment of the present invention;
FIG. 2 is a schematic view of a navigation interface according to one embodiment of the present invention;
FIG. 3 is a flowchart of a system operation program of the surgical system according to one embodiment of the present invention;
FIG. 4 is a detailed flowchart of step S17 of the system operation program;
FIG. 5 is another flowchart of the system operation program of the surgical system according to one embodiment of the present invention;
FIG. 6 is a top view of a surgical tool and a cannular scope according to one embodiment of the present invention; and
FIG. 7 is a detailed flowchart of step S27 of the system operation program.

### DETAILED DESCRIPTION OF THE EXEMPLARY EMBODIMENTS

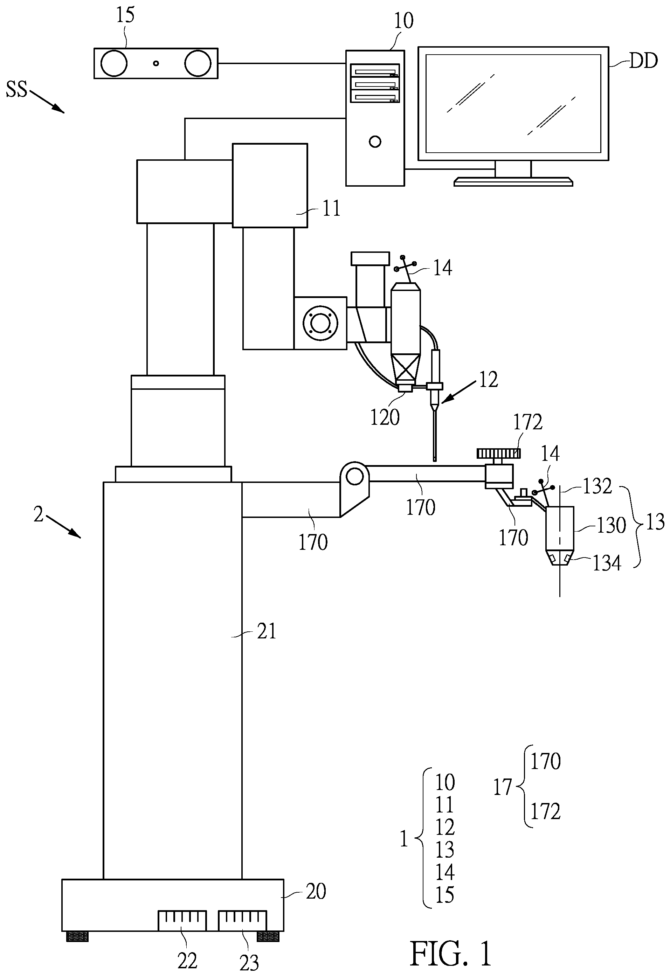

FIG. 1 is a schematic view of configurations of a surgical system and a decompression system according to one embodiment of the present invention. Referring to FIG. 1, one embodiment of the present invention provides a surgical system SS, and the surgical system SS includes a decompression system 1 and a display device DD. The decompression system 1 may include a surgical navigation module 10, a robotic arm 11, a surgical tool 12, a cannular scope 13, a plurality of detection markers 14, and a navigation image capture device 15.

In the embodiment of FIG. 1, the decompression system 1 is essentially deployed around a mobile trolley 2, and the mobile trolley 2 may include a base 20 and a support column 21. The bottom of the base 20 is provided with a plurality of moving mechanisms (for example, wheels) and a platform for disposing the support column 21. The top of the support column 21 is provided with the robotic arm 11, and the base 20 and the support column 21 together serve as a fixing part for supporting the robotic arm 11. The robotic arm 11 may connect with a parallel-type machine device, such as an end effector based on a Stewart platform having multiple degrees of freedom and driven by multiple motors. Through an adapter, the robotic arm 11 may grip the surgical tool 12, such as a drill, a trocar, or a saw blade. The surgical tool 12 is disposed on the robotic arm 11 and has a force sensor 120. The force sensor 120 may be, for example, a six-axis force sensor used to measure current force data on the surgical tool 12, and the current force data includes a force value, a force direction, etc. The decompression system 1 further includes a first operation interface electrically connected to the surgical navigation module 10. For example, a first pedal 22 extending from the bottom of the base 20 is used to control the surgical tool 12 to enter or exit a drive mode. For example, when a user (e.g., a surgeon) treads on the first pedal 22, a drill bit or a saw blade can be controlled to start rotating.

In certain embodiments, the robotic arm 11 may adopt a parallel-type machine device having six degrees of freedom, and the parallel-type machine device contains six sets of actuation units and six sets of corresponding limbs. Each of the actuation units may include a motor, a coupling device, a lead screw, and a slide rod. When the actuation units drive the limbs, a surgical instrument on the adapter will be correspondingly moving to a predetermined position/direction. The above-mentioned parallel-type machine device adopts a design of the Stewart platform that is well known in the art, therefore details of the platform will not be recited again. In addition to the above-mentioned parallel-type machine device, the robotic arm 11 may be connected to a serial-type machine device. Here, the robotic arm 11 can be operated by the user. During operation, the user is guided to move the robotic arm 11 along a specific path, thereby accurately controlling the position and direction of the surgical tool 12.

On the other hand, the support column 21 of the mobile trolley 2 further includes a movable support arm 17. The movable support arm 17 has a plurality of link structures 170 that are pivotally connected to each other, and a cannular scope 13 is provided at the terminal link structure 170 of the movable support arm 17. The link structures 170 can be fixed by a locking mechanism 172 (e.g., a knob), such that a movable portion of the movable support arm 17 is fixed along with the cannular scope 13. The decompression system 1 further includes a second operation interface electrically connected to the surgical navigation module 10. For example, a second pedal 23 that extends from the bottom of the base 20 is used to lock or release the movable support arm 17. For example, when the user treads on the second pedal 23, the movable support arm 17 can be released, and the user can manually move the movable support arm 17, such that the movable support arm 17 as well as the cannular scope 13 can be moved together to a specific position. When the user releases the second pedal 23, the movable support arm 17 is locked and stays in a stationary state.

The cannular scope 13 is disposed on the movable support arm 17, and the cannular scope 13 includes a lens barrel 130 and at least one camera 134 disposed in the lens barrel 130. For example, the lens barrel 130 can be a cylindrical shell extending along a cannula axis 132, and the camera 134 can be disposed on an inner wall of the cylindrical shell. When the cannular scope 13 is placed into an incision during a surgery, the camera 134 can obtain images of a surgical site for the surgeon to view, thereby allowing the surgeon to perform subtle and precise operations in a very small working space and avoiding damage to nerve tissues or unnecessary destruction to normal tissues. Furthermore, one or more light sources can also be disposed in the lens barrel 130 for lighting the surgical site. Moreover, after the cannular scope 13 is placed above the surgical site, the locking mechanism 172 is operated to fix the movable portion of the movable support arm 17.

A plurality of detection markers 14 are respectively disposed on the surgical tool 12, the cannular scope 13, and a patient. By using an existing positioning method, such as optical positioning, electromagnetic positioning, or inertial positioning to detect the detection markers 14, the directions and positions of the surgical tool 12 and the cannular scope 13 relative to the surgical site of the patient can be measured. In addition, the detection markers 14 may include, e.g., a plurality of markers for emitting electromagnetic signals, sound waves, heat, or other perceivable signals, and may be respectively installed on the surgical tool 12, the cannular scope 13, and the patient in specific directions and angles. In the embodiment of the present invention, an optical positioning manner is adopted. Therefore, the detection markers may be reflective balls or marking devices that actively generate perceivable signals. Moreover, multiple detection markers can also be provided near the surgical site (for example, a dynamic reference frame fixed near the surgical site).

The surgical navigation module 10 may be a computing device including a processor, a memory, an input-output interface, and an image processor. The surgical navigation module 10 may be electrically connected to the robotic arm 11, the surgical tool 12, the cannular scope 13, the navigation image capture device 15, and the display device DD. The navigation image capture device 15 may be such as a pair of cameras configured to capture images of the detection markers 14.

The decompression system 1 may further include one or more power supplies for supplying power to, e.g., the surgical navigation module 10, the robotic arm 11, the surgical tool 12, the cannular scope 13, the plurality of detection markers 14, and the navigation image capture device 15.

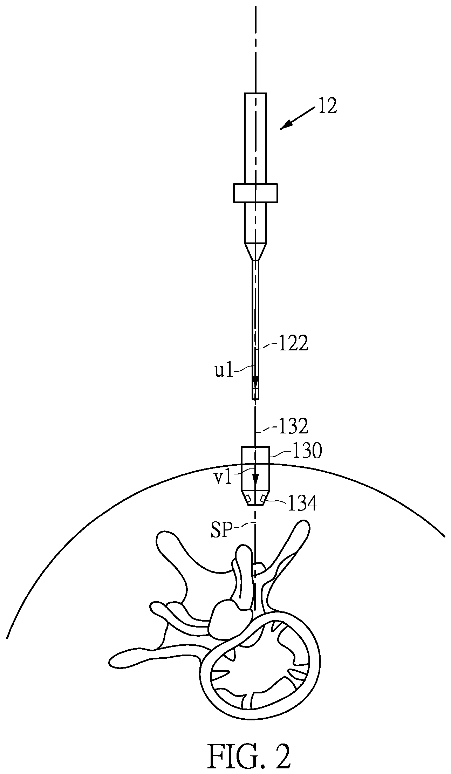

The display device DD may be a display device having a touch control function. As shown in FIG. 2, the display device DD can be used for the user to plan a predetermined surgical path SP, and display an anatomy model of the patient, the predetermined surgical path SP, and the images of the surgical site captured by the camera 134.

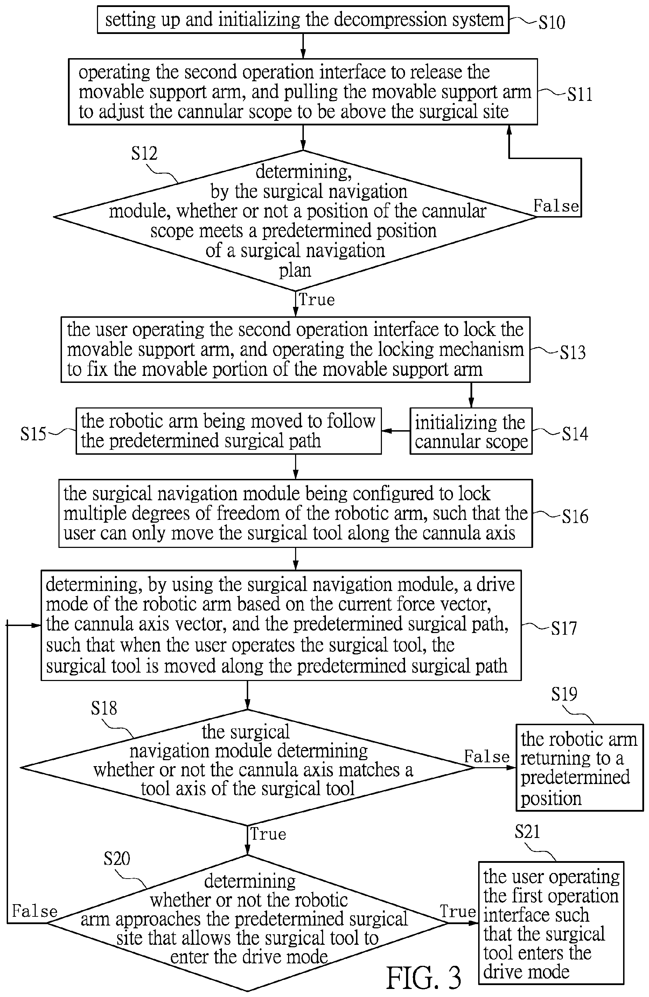

Referring to FIG. 3, FIG. 3 is a flowchart of a system operation program of the surgical system according to one embodiment of the present invention. As shown in FIG. 3, the system operation program includes the following steps.

Step S10: setting up and initializing the decompression system.

In this step, the mobile trolley 2 can be moved to a side of an operating table, and the power can be turned on to initialize the decompression system 1. During the initialization process, the surgical navigation module 10 may be configured to confirm power statuses and communication statuses of the robotic arm 11, the surgical tool 12, the cannular scope 13, and the navigation image capture device 15, and check if the aforementioned members can operate normally. In addition, the navigation image capture device 15 is also utilized for checking whether or not the detection markers 14 can be detected and located at correct positions, such as whether or not the detection markers 14 are disposed on the surgical tool 12, the cannular scope 13, and the patient.

After the system operates normally, the system operation program proceeds to step S11: operating the second operation interface to release the movable support arm 17, and pulling the movable support arm 17 to adjust the cannular scope 13 to be above the surgical site. For example, the cannular scope 13 can be placed in the incision.

Step S12: determining, by the surgical navigation module 10, whether or not a position of the cannular scope 13 meets a predetermined position of a surgical navigation plan.

In detail, the plurality of detection markers 14 can be disposed near the surgical site, and then a computed tomography (CT) or magnetic resonance imaging (MRI) scanning can be performed around the surgical site of the patient. The surgical navigation module 10 can obtain the images of the detection markers 14 near the surgical site through the navigation image capture device 15, combine the pre-obtained CT images or MRI images to establish a three-dimensional anatomy model of the surgical site and internal skeleton, and mark the positions of the detection markers in the three-dimensional anatomy model. The established three-dimensional anatomy model can be processed and rendered by the image processor, and then displayed in a navigation interface on the display device DD.

Then, the user can generate the surgical navigation plan (including setting a predetermined surgical site, the predetermined position, and the predetermined surgical path SP in the three-dimensional anatomy model displayed on the display device DD). The upside of the predetermined surgical site can be identified as the predetermined position of the surgical navigation plan. The predetermined position is a suitable position for the cannular scope to be fixed. In addition, the user can also plan the predetermined surgical path SP, for example, based on a position of the spinal disc that compresses nerves. Moreover, when planning of the predetermined surgical path SP is completed, the surgical navigation module 10 can further simultaneously display the predetermined surgical site, the predetermined position, and the predetermined surgical path SP in the navigation interface, so as to indicate to the user the surgical site (i.e. the region corresponding to the predetermined surgical site and on which a surgery is to be performed) and allow the cannular scope 13 to move along the predetermined surgical path SP and be located above the surgical site (i.e., at the predetermined position of the surgical navigation plan), thereby assisting the user to steadily push the surgical tool 12 along the predetermined surgical path SP during the surgery.

Therefore, in step S12, the surgical navigation module 10 can determine whether or not the position of the cannular scope 13 meets the predetermined position of the surgical navigation plan, and display a determination result through the display device DD.

In response to the surgical navigation module 10 determining that the position of the cannular scope 13 does not meet the predetermined position of the surgical navigation plan, the display device DD prompt the user to repeat step S11.

In response to the surgical navigation module 10 determining that the position of the cannular scope 13 meets the predetermined position of the surgical navigation plan, the display device DD prompt the user to proceed to step S13.

Step S13: the user operating the second operation interface to lock the movable support arm 17, and operating the locking mechanism 172 to fix the movable portion of the movable support arm 17. For example, the user can release the second pedal 23 and tighten the knob to fix the movable support arm 17, such that the cannular scope 13 is also fixed relative to the movable support arm 17.

Step S14: initializing the cannular scope 13. In this step, the surgical navigation module 10 can turn on the camera 134 and the light source, and enlarge an image captured by the camera 134 and display the image on the display device DD.

Step S15: the robotic arm 11 being moved to follow the predetermined surgical path SP. In this step, the robotic arm 11 connected to the surgical tool 12 is operated by the user such that the surgical tool 12 is positioned on the predetermined surgical path SP aligned with the cannula axis 132 and does not contact other parts. For example, when the cannular scope 13 is disposed at the predetermined position of the surgical navigation plan, the predetermined surgical path SP may be such as a virtual path that coincides with the cannula axis 132. When the surgical tool 12 is located on the predetermined surgical path SP aligned with the cannula axis 132, the system operation program proceeds to step S16.

Step S16: the surgical navigation module 10 being configured to lock multiple degrees of freedom of the robotic arm 11, such that the user can only move the surgical tool 12 along the cannula axis 132. For example, the cannula axis 132 can be taken as a Z-axis, and most degrees of freedom of the robotic arm 11 are locked so that only movement of the surgical tool 12 in the Z-axis is allowed, thereby improving operation stability of the surgical tool 12.

After step S16 is performed, the robotic arm 11 enters a standby mode, and waits for the user to hold and move the robotic arm 11.

Step S17: determining, by using the surgical navigation module 10, a drive mode of the robotic arm 11 based on the current force vector, the cannula axis vector, and the predetermined surgical path SP, such that when the user operates the surgical tool 12, the surgical tool 12 is moved along the predetermined surgical path SP.

In detail, the surgical navigation module 10 may be configured to calculate the force component of the current force vector in a direction along the cannula axis vector, and determine whether or not the force component is greater than a first force threshold.

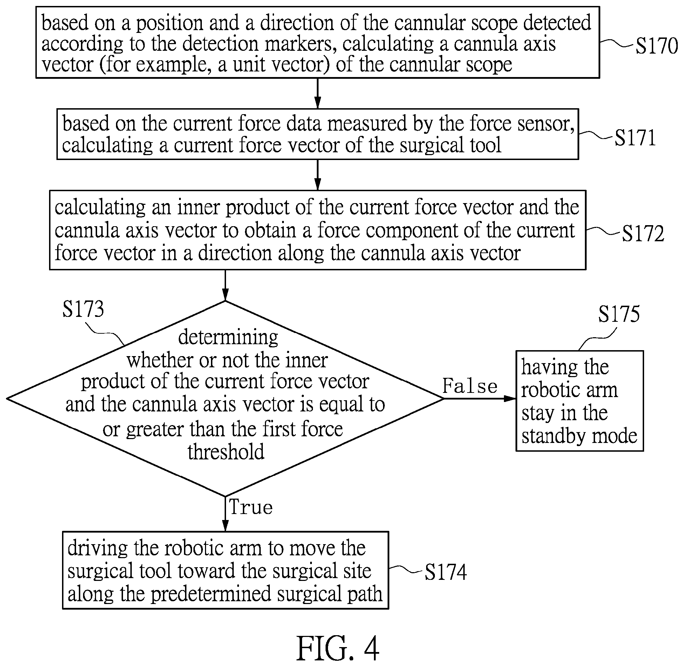

FIG. 4 is a detailed flowchart of step S17. Referring to FIG. 4, step S17 may further include performing the following steps by using the surgical navigation module 10.

Step S170: based on a position and a direction of the cannular scope 13 detected according to the detection markers 14, calculating a cannula axis vector u1 (for example, a unit vector) of the cannular scope 13.

Step S171: based on the current force data measured by the force sensor 120, calculating a current force vector v1 of the surgical tool 12.

Step S172: calculating an inner product of the current force vector v1 and the cannula axis vector u1 to obtain a force component of the current force vector v1 in a direction along the cannula axis vector u1.

Step S173: determining whether the inner product of the current force vector v1 and the cannula axis vector u1 is equal to or greater than the first force threshold.

In response to determining in step S173 that the force component is equal to or greater than the first force threshold, the system operation program proceeds to step S174, and step S174 includes driving the robotic arm 11 to move the surgical tool 12 toward the surgical site along the predetermined surgical path SP.

In response to determining in step S173 that the force component is not greater than the first force threshold, the system operation program proceeds to step S175, and step S175 includes having the robotic arm 11 stay in the standby mode.

Therefore, in the system operation program, after the surgical tool 12 is operated and positioned on the predetermined surgical path SP aligned with the cannula axis, the surgical navigation module 10 can determine whether or not the robotic arm 11 should be driven to move the surgical tool 12 along the predetermined surgical path SP based on the current force vector v1, the cannula axis vector u1, and the predetermined surgical path SP. Furthermore, by setting the first force threshold, it can be ensured that the surgical tool 12 can be moved only when a force applied on the surgical tool 12 is greater than a certain level. For example, the first force threshold is set as fth. When the current force vector v1 is a downward (for example, a negative Z direction) vector, and the cannula axis vector u1 is also a downward vector, the inner product (represented by |u1|*|v1|*cos(0 degrees)) of the two vectors has a positive value. If the positive value is greater than fth, then the condition of the force component being greater than the first force threshold is met.

Reference is further made to FIG. 3. The system operation program further includes step S18 in which the surgical navigation module 10 determines whether or not the cannula axis 132 matches a tool axis 122 of the surgical tool 12. For example, when the surgical tool 12 is a drill bit, the tool axis 122 is the axis of the drill bit. Through this safety mechanism, it ensures that the surgical tool 12 and the cannula axis 132 are still in an aligned state when the robotic arm 11 moves.

In response to determining in step S18 that the cannula axis 132 does not match the tool axis 122, the system operation program proceeds to step S19, which includes resetting the robotic arm 11. In step S19, the robotic arm 11 returns to an initial position. The initial position may be, for example, a starting point before the system operation program determines whether the force component is greater than the first force threshold, and before the robotic arm 11 starts moving toward the surgical site.

In response to determining in step S18 that the cannula axis 132 matches the tool axis 122, the system operation program proceeds to step S20, which includes determining whether or not the robotic arm 11 approaches the predetermined surgical site that allows the surgical tool 12 to enter the drive mode.

Similarly, the predetermined surgical site can be planned along with the predetermined surgical path in the three-dimensional anatomy model displayed on the display device DD, and the surgical navigation module 10 can further display the predetermined surgical site in the navigation interface to guide the user. For example, the surgical system allows the user to control rotating of the drill bit only after the drill bit touches the predetermined surgical site.

In response to determining in step S20 that the robotic arm 11 has not reached the predetermined surgical site, step S17 is repeated.

In response to determining in step S20 that the robotic arm 11 has reached the predetermined surgical site, the system operation program proceeds to step S21: the user operating the first operation interface such that the surgical tool 12 enters the drive mode. For example, the user can operate the surgical tool 12 (e.g., a drill bit) and tread on the first pedal 22 to initiate rotation of the drill bit.

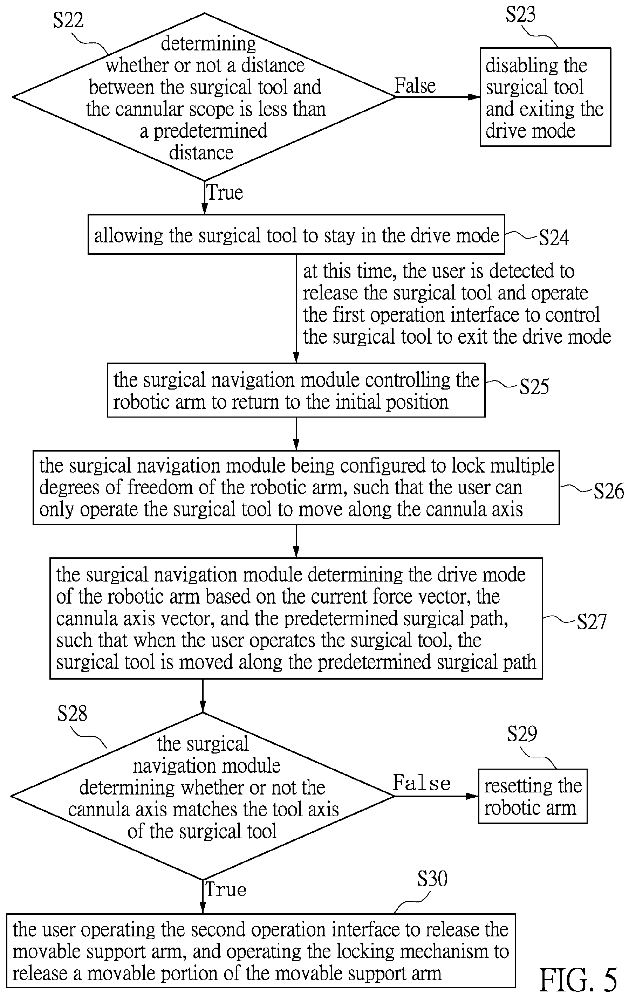

Reference is made to FIG. 5, which is another flowchart of the system operation program of the surgical system according to one embodiment of the present invention.

When step S21 is executed, step S22 is simultaneously executed, and step S22 includes determining whether or not a distance between the surgical tool 12 and the cannular scope 13 is less than a predetermined distance.

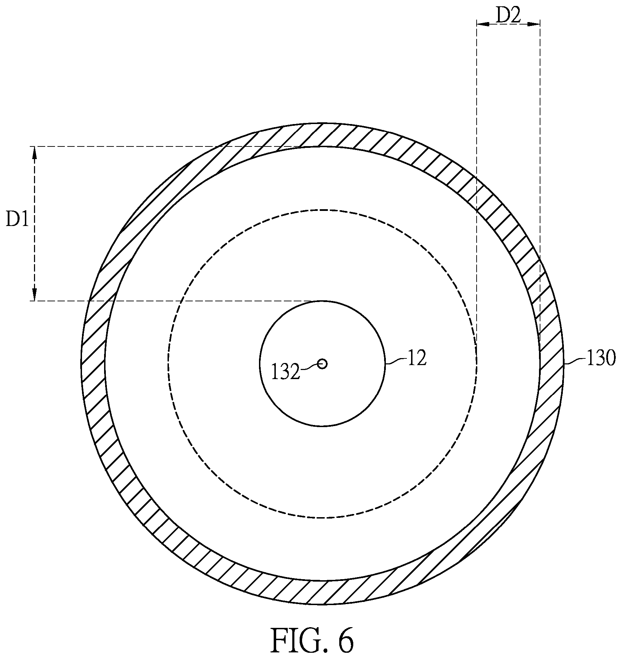

Referring to FIG. 6, FIG. 6 is a top view of the surgical tool and the cannular scope according to one embodiment of the present invention. As shown in the figure, when the robotic arm 11 reaches the predetermined surgical site, part of the surgical tool 12 will pass through the lens barrel 130 (e.g., a cylindrical shell extending along the cannula axis 132). At this time, it is necessary to determine whether or not a distance D1 between a portion of the surgical tool 12 located in the lens barrel 130 and an inner wall of the lens barrel 130 is less than a predetermined distance (e.g., a predetermined safety distance D2), thereby preventing the surgical tool 12 from colliding with the lens barrel 130 and from damaging the camera 134. Furthermore, as the surgical tool 12 is too close to the cannular scope 13, the surgical tool 12 is prone to collide with the cannular scope 13, therefore causing shaking of images captured by the camera 134 and finally inducing uncertainty during the surgery. By means of determining whether or not the distance D1 is less than the predetermined distance, the uncertainty during the surgery can be reduced.

In response to determining in step S22 that the distance between the surgical tool 12 and the cannular scope 13 is less than the predetermined distance, the system operation program proceeds to step S23, which includes disabling the surgical tool 12 and exiting the drive mode. For example, power to the surgical tool 12 is compulsorily turned off and operation of the surgical tool 12 is stopped.

In response to determining in step S22 that the distance between the surgical tool 12 and the cannular scope 13 is not less than the predetermined distance, the system operation program proceeds to step S24, which includes allowing the surgical tool 12 to stay in the drive mode.

In the system operation program, when the system detects that the user releases the surgical tool 12 and operates the first operation interface to control the surgical tool 12 to exit the drive mode, the system operation program proceeds to step S25: the surgical navigation module 10 controlling the robotic arm 11 to return to the initial position.

Step S26: the surgical navigation module 10 being configured to lock multiple degrees of freedom of the robotic arm 11, such that the user can only operate the surgical tool 12 to move along the cannula axis 132. It should be noted that, the sequence of performing steps S26 and S25 can also be adjusted according to the requirement of the user. For example, step S26 is performed before step S25.

After step S26, the robotic arm 11 enters the standby mode and waits for the user to hold and move the robotic arm 11.

Step S27: the surgical navigation module 10 determining the drive mode of the robotic arm 11 based on the current force vector v1, the cannula axis vector u1, and the predetermined surgical path SP, such that when the user operates the surgical tool 12, the surgical tool 12 is moved along the predetermined surgical path SP.

In detail, the surgical navigation module 10 may be configured to calculate the force component of the current force vector v1 in a direction along the cannula axis vector u1, and determine whether or not the force component is less than the second force threshold.

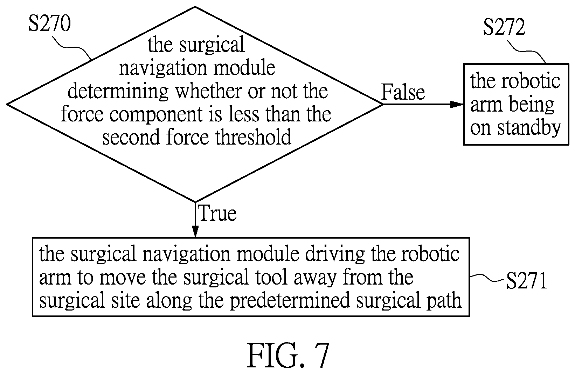

FIG. 7 is a detailed flow chart of step S27. Reference is made to FIG. 7, in which step S27 may further include executing the following steps by using the surgical navigation module 10.

Step S270: the surgical navigation module 10 determining whether or not the force component is less than the second force threshold.

In response to determining in step S270 that the force component is less than the second force threshold, the system operation program proceeds to step S271 in which the surgical navigation module 10 drives the robotic arm 11 to move the surgical tool 12 away from the surgical site along the predetermined surgical path SP.

In response to determining in step S270 that the force component is not less than the second force threshold, the system operation program proceeds to step S272 in which the robotic arm 11 is on standby.

For example, the second force threshold is -fth. When the current force vector v1 is an upward (for example, a positive Z direction) vector, and the cannula axis vector u1 is a downward vector, then the inner product (represented by [u1|*|v1|*cos(180 degrees)) of the two vectors has a negative value. If the negative value is less than -fth (i.e., the absolute value of the inner product is greater than |-fth|), a condition of the force component being less than the second force threshold is met, indicating that a force greater than a certain level is present at the time the robotic arm 11 is operated to move upward.

Reference is further made to FIG. 5. The system operation program further includes step S28 in which the surgical navigation module 10 determines whether or not the cannula axis 132 matches the tool axis 122 of the surgical tool 12. For example, when the surgical tool 12 is a drill bit, the tool axis 122 is an axis of the drill bit. This safety mechanism ensures that the surgical tool 12 and the cannula axis 132 are still in an aligned state when the robotic arm 11 moves.

In response to determining in step S28 that the cannula axis 132 does not match the tool axis 122, the system operation program proceeds to step S29 which includes resetting the robotic arm 11. Step S29 is similar to step S19 and allows the robotic arm 11 to return to the predetermined position.

In response to determining that the cannula axis 132 matches the tool axis 122 in step S28, the system operation program proceeds to step S30 in which the user operates the second operation interface to release the movable support arm 17, and operates the locking mechanism 172 to release a movable portion of the movable support arm 17. For example, the user can tread on the second pedal 23 and loosen the knob, thereby allowing the movable support arm 17 to be movable, and the cannular scope 13 also is movable relative to the movable support arm 17. Afterwards, the cannular scope 13 can be removed and the movable support arm 17 can be folded.

After step S30, the camera 134 and the light source of the cannular scope 13 can be turned off, and then the surgical navigation module 10 can be turned off.

### [Beneficial Effects of the Embodiments]

One of the beneficial effects of the present invention is that, the surgical system and the decompression system provided by the present invention are designed with multiple safety mechanisms, including locking degrees of freedom of the robotic arm under various specific circumstances, determining the drive mode of the robotic arm based on the current force vector, the cannula axis vector, and the predetermined surgical path, determining whether or not the cannula axis matches the tool axis of the surgical tool, and determining whether or not the distance between the surgical tool and the cannular scope is less than the safe distance, etc. Accordingly, it can be ensured that the surgical tool can be moved only when a force on the surgical tool is greater than a certain level, thereby improving the operation stability of the surgical tool. Furthermore, as the surgical tool is too close to the cannular scope, the surgical tool is prone to collide with the cannular scope, therefore causing shaking of the images captured by the camera and finally inducing uncertainty during the surgery. By means of determining whether or not the distance is less than the predetermined distance, the surgical tool is prevented from colliding with the lens barrel and from damaging the camera, and the uncertainty during the surgery can be reduced.

Moreover, the surgical system and the decompression system provided by the present invention apply the optical navigation technology to the cannular scope and the surgical tool, thereby allowing surgeons to more conveniently learn the spatial relationship between the cannular scope and the surgical tool. By real-time monitoring the cannula axis of the cannular scope and the tool axis of the surgical tool, surgery safety can be improved. When the surgical tool approaches the inner wall of the lens barrel, a power-off mechanism is triggered to improve safety of using the surgical tool.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated.

## Claims

1. A surgical system (SS), **characterized by** comprising:
a surgical navigation module (10);
a robotic arm (11) electrically connected to the surgical navigation module (10);
a surgical tool (12) disposed on the robotic arm (11), wherein the surgical tool (12) includes a force sensor (120), and the force sensor (120) is configured to measure current force data on the surgical tool (12);
a cannular scope (13) disposed on a movable support arm (17), wherein the cannular scope (13) has a cannula axis (132) and at least one camera (134);
a plurality of detection markers (14) respectively disposed on the surgical tool (12), the cannular scope (13), and a patient;
a navigation image capture device (15) electrically connected to the surgical navigation module (10) and configured to capture images of the plurality of detection markers (14); and
a display device (DD) electrically connected to the surgical navigation module (10) for allowing a user to plan a predetermined surgical path (SP), wherein the display device (DD) displays an anatomy model of the patient, the predetermined surgical path (SP), and an image of a surgical site captured by the at least one camera (134);
wherein the surgical navigation module (10) is configured to calculate and obtain a cannula axis vector (u1) of the cannular scope (13) based on the plurality of detection markers (14), and calculate and obtain a current force vector (v1) of the surgical tool (12) based on the current force data;
wherein, in a system operation program, after the surgical tool (12) is located on the predetermined surgical path (SP) aligned with the cannula axis (132), the surgical navigation module (10) is configured to determine a drive mode of the robotic arm (11) based on the current force vector (v1), the cannula axis vector (u1), and the predetermined surgical path (SP), so that when the user operates the surgical tool (12), the surgical tool (12) is moved along the predetermined surgical path (SP).

2. The surgical system (SS) according to claim 1, further comprising a first operation interface and a second operation interface, and the first operation interface and the second operation interface both being electrically connected to the surgical navigation module (10), wherein the first operation interface is used to control the surgical tool (12) to enter or exit the drive mode, and the second operation interface is used to lock or release the movable support arm (17).

3. The surgical system (SS) according to claim 2, wherein the surgical navigation module (10) is configured to calculate a force component of the current force vector (v1) in a direction along the cannula axis vector (u1), and determine whether or not the force component is greater than a first force threshold;
wherein, in response to determining that the force component is greater than the first force threshold, the surgical navigation module (10) drives the robotic arm (11) to move the surgical tool (12) toward the surgical site along the predetermined surgical path (SP).

4. The surgical system (SS) according to claim 3, wherein the surgical navigation module (10) is further configured to determine whether or not the force component is less than a second force threshold;
wherein, in response to the force component being less than the second force threshold, the surgical navigation module (10) drives the robotic arm (11) to move the surgical tool (12) away from the surgical site along the predetermined surgical path (SP).

5. The surgical system (SS) according to claim 4, wherein the surgical navigation module (10) calculates an inner product of the current force vector (v1) and the cannula axis vector (u1) to obtain the force component.

6. The surgical system (SS) according to claim 2, wherein, in the system operation program, the surgical navigation module (10) is further configured to determine whether or not the robotic arm (11) reaches a predetermined surgical site that allows the surgical tool (12) to enter the drive mode;
wherein, in response to determining that the robotic arm (11) has reached the predetermined surgical site, when the first operation interface is operated so that the surgical tool (12) enters the drive mode, the surgical navigation module (10) determines whether or not a distance (D1) between the surgical tool (12) and the cannular scope (13) is less than a predetermined distance;
wherein, in response to determining that the distance (D1) between the surgical tool (12) and the cannular scope (13) is less than the predetermined distance, the surgical tool (12) is disabled and exits the drive mode; and
wherein, in response to determining that the distance (D 1) between the surgical tool (12) and the cannular scope (13) is greater than or equal to the predetermined distance, the surgical tool (12) is allowed to stay in the drive mode.

7. The surgical system (SS) according to claim 1, wherein, in the system operation program, in response to the cannular scope (13) being disposed above the surgical site and the surgical tool (12) being located on the predetermined surgical path (SP) aligned with the cannula axis (132), the surgical navigation module (10) is configured to lock multiple degrees of freedom of the robotic arm (11), so that the user can only operate the surgical tool (12) to move along the cannula axis (132).

8. The surgical system (SS) according to claim 2, wherein, in response to detecting that the user releases the surgical tool (12) in the system operation program and the first operation interface is operated to control the surgical tool (12) to exit the drive mode, the surgical navigation module (10) controls the robotic arm (11) to return to an initial position.

9. The surgical system (SS) according to claim 8, wherein, in the system operation program, the surgical navigation module (10) is further configured to determine whether or not the cannula axis (132) matches a tool axis (122) of the surgical tool (12);
wherein, in response to determining that the cannula axis (132) does not match the tool axis (122), the robotic arm (11) is reset to the initial position.

10. The surgical system (SS) according to claim 1, wherein the movable support arm (17) further includes a locking mechanism (172), and wherein, in the system operation program, after the cannular scope (13) is disposed above the surgical site, the locking mechanism (172) is operated to fix a movable portion of the movable support arm (17).

11. A decompression system (1), **characterized by** comprising:
a surgical navigation module (10);
a robotic arm (11) electrically connected to the surgical navigation module (10);
a surgical tool (12) including a force sensor (120), wherein the force sensor (120) is configured to measure current force data on the surgical tool (12);
a cannular scope (13) having a cannula axis (132);
a plurality of detection markers (14) respectively disposed on the surgical tool (12), the cannular scope (13), and a patient; and
an image capture device electrically connected to the surgical navigation module (10) and configured to capture images of the plurality of detection markers (14);
wherein the surgical navigation module (10) is configured to calculate and obtain a cannula axis vector (u1) of the cannular scope (13) based on marker positions of the plurality of detection markers (14), and calculate and obtain a current force vector (v1) of the surgical tool (12) based on the current force data;
wherein, in a system operation program, after the surgical tool (12) is operated and located on the predetermined surgical path (SP) aligned with the cannula axis (132), the surgical navigation module (10) is configured to determine a drive mode of the robotic arm (11) based on the current force vector (v1), the cannula axis vector (u1), and the predetermined surgical path (SP), so that when the user operates the surgical tool (12), the surgical tool (12) is moved along the predetermined surgical path (SP).
